# FASCICULE DE BREVET EUROPEEN

(11) **EP 1 090 628 B1**
(45) Date de publication et mention de la délivrance du brevet: **09.11.2005**
(21) Numéro de dépôt: 00402461.8
(22) Date de dépôt: 07.09.2000
(51) Int. Cl.: A61K 7/48, A61K 7/075

(54) **Utilisation du lycopène dans des compositions destinées à traiter les signes cutanés du vieillissement**
Verwendung von Lycopin in Zusammensetzungen zur Behandlung von Hautalterungs-Erscheinungen
Use of lycopene in compositions for treating ageing skin symptoms

(30) Priorité: 07.10.1999 FR 9912507; 27.03.2000 FR 0003849
(43) Date de publication de la demande: 11.04.2001
(73) Titulaire: L'OREAL, 75008 Paris (FR)
(72) Inventeur: Lorant, Raluca, 94320 Thiais (FR); Breton, Lionel, 78000 Versailles (FR); Liviero, Christel, 75008 Paris (FR)
(74) Mandataire: Allab, Myriam

(56) Documents cités:
- EP-A- 0 659 402
- WO-A-97/47278
- US-A- 5 290 605
- US-A- 5 895 652
- US-A- 5 925 348

## Description

L'invention se rapporte à l'utilisation du lycopène dans ou pour la préparation d'une composition destinée à traiter, de manière préventive et/ou curative, les signes cutanés du vieillissement. En particulier, le lycopène ou la composition sont destinés à inhiber la dégradation de la peau et/ou des muqueuses par l'inhibition des collagénases. L'invention a également pour objet un procédé de traitement cosmétique de la peau et/ou des muqueuses.

La peau humaine est constituée de deux compartiments à savoir un compartiment superficiel, l'épiderme, et un compartiment profond, le derme.

L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.

Le derme fournit à l'épiderme un support solide. C'est également son élément nourricier. Il est principalement constitué de fibroblastes et d'une matrice extracellulaire composée elle-même principalement de collagène, d'élastine et d'une substance, dite substance fondamentale, composants synthétisés par le fibroblaste. On y trouve aussi des leucocytes, des mastocytes ou encore des macrophages tissulaires. Il est également traversé par des vaisseaux sanguins et des fibres nerveuses. Dans une peau normale, c'est à dire non pathologique ni cicatricielle, le fibroblaste est à l'état quiescent, c'est à dire non prolifératif, peu actif d'un point de vue métabolique et non mobile.

Ce sont les fibres de collagène qui assurent la solidité du derme. Les fibres de collagène sont constituées de fibrilles scellées les unes aux autres, formant ainsi plus de dix types de structures différentes. La solidité du derme est en grande partie due à l'enchevêtrement des fibres de collagène tassées les unes contre les autres en tous sens. Les fibres de collagène participent à l'élasticité et à la tonicité de la peau et/ou des muqueuses.
Les fibres de collagènes sont constamment renouvelées mais ce renouvellement diminue avec l'âge ce qui entraîne un amincissement du derme. Cet amincissement du derme est également dû à des causes pathologiques comme par exemple l'hypersécrétion d'hormones corticoïdes, certaines pathologies ou encore des carences vitaminiques (cas de la vitamine C dans le scorbut). Il est également admis que des facteurs extrinsèques comme les rayons ultraviolets, le tabac ou certains traitements (Glucocorticoïdes, vitamine D et dérivés par exemple) ont également un effet sur la peau et sur son taux de collagène.

Cependant, divers facteurs entraînent la dégradation du collagène, avec toutes les conséquences que l'on peut envisager sur la structure et/ou la fermeté de la peau et/ou des muqueuses.

Bien que très résistantes, les fibres de collagène sont sensibles à certaines enzymes appelées collagénases. Une dégradation des fibres de collagène entraîne l'apparence de peau molle et ridée que l'être humain, préférant l'apparence d'une peau lisse et tendue, cherche depuis toujours à combattre.

Les collagénases font partie d'une famille d'enzymes appelées métalloprotéinases (MMPs) qui sont elles-mêmes les membres d'une famille d'enzymes protéolytiques (endoprotéases) qui possèdent un atome de zinc coordonné à 3 résidus cystéine et une méthionine dans leur site actif et qui dégradent les composants macromoléculaires de la matrice extracellulaire et des lames basales à pH neutre (collagène, élastine, etc....). Très largement répandues dans le monde vivant, ces enzymes sont présentes, mais faiblement exprimées, dans des situations physiologiques normales comme la croissance des organes et le renouvellement des tissus.

Leur surexpression chez l'homme et leur activation sont cependant liées à de nombreux processus, parfois pathologiques, qui impliquent la destruction et le remodelage de la matrice. Cela entraîne soit une résorption non contrôlée de la matrice extracellulaire, soit inversement l'installation d'un état de fibrose.

La famille des métalloprotéinases est constituée de plusieurs groupes bien définis basés sur leurs ressemblances en terme de structure et de spécificité de substrat (voir Woessner J. F., Faseb Journal, vol. 5, 1991, 2145). Parmi ces groupes, on peut citer les collagénases destinées à dégrader les collagènes fibrillaires (MMP-1 ou collagénase interstitielle, MMP-8 ou collagénase de neutrophile, MMP-13 ou collagénase 3), les gélatinases qui dégradent le collagène de type IV ou toute forme de collagène dénaturé (MMP-2 ou gélatinase A (72 kDa), MMP-9 ou gélatinase B (92 kDa)), les stromélysines (MMP-3) dont le large spectre d'activité s'adresse aux protéines de la matrice extracellulaire telles que les glycoprotéines (fibronectine, laminine), les protéoglycannes, etc., ou encore les métalloprotéinases membranaires.
L'exposition prolongée aux rayonnements ultraviolets, particulièrement aux rayonnements ultraviolets de type A et/ou B, a pour effet une stimulation de l'expression des collagénases, particulièrement de la MMP-1. C'est là une des composantes du vieillissement cutané photo-induit.

Par ailleurs à la ménopause, les principales modifications concernant le derme sont une diminution du taux de collagène et de l'épaisseur dermique. Cela entraîne chez la femme ménopausée un amincissement de la peau et/ou des muqueuses. La femme ressent alors une sensation de "peau sèche" ou de peau qui tire et l'on constate une accentuation des fines rides et ridules de surface. La peau présente un aspect rugueux à la palpation. Enfin la peau présente une souplesse diminuée.

On comprend alors à la lecture de ce qui précède l'importance du collagène dans la structure des tissus, particulièrement de la peau et/ou des muqueuses, et l'importance qu'il y a à combattre sa dégradation pour ainsi lutter contre le vieillissement qu'il soit chronobiologique ou photo-induit et ses conséquences, l'amincissement du derme et/ou la dégradation des fibres de collagène ce qui entraînent l'apparence de peau molle et ridée.

Un des buts de la présente invention est donc de pouvoir disposer d'un produit nouveau qui présente un effet inhibiteur des collagénases et si possible pas d'effets secondaires notables.

Or, de manière surprenante et inattendue la demanderesse a maintenant découvert que le lycopène présente une activité inhibitrice de l'activité des collagénases.

Le lycopène est un pigment naturel que l'on trouve dans les fruits mûrs, particulièrement dans la tomate. Il appartient à la famille des caroténoïdes et sa structure est proche de celle du β-carotène.
Le rôle du lycopène dans la maturation des fruits est connu dans l'art antérieur. Le lycopène est utilisé dans des compositions à activité bronzante pour son rôle sur la synthèse de mélanine (WO 97/47278), dans des compositions destinées au traitement de la chevelure et/ou de l'acné pour son activité sur les 5α-réductases (JP-2940964) ou encore comme agent anti-radicalaire (JP-A-8-283136).

A la connaissance de la demanderesse l'activité inhibitrice de l'activité des collagénases du lycopène n'a jamais été décrite.

US 5 925 348 décrit des compositions destinée à lutter contre le vieillissement cutané dans lesquelles le principe actif est un extrait de Lotus sacré. Celui-ci intervient grâce à la présence d'une L-isoaspartyl méthyltransférase.
US 5 895 652 concerne des suppléments diététiques assurant un bon fonctionnement du métabolisme humain; les compositions contiennent des mélanges complexes de vitamines, minéraux, extraits de plante, enzymes et régulateurs de pH. US 5 290 605 vise une boisson à absorber concomitamment à l'exposition au soleil, et comprenant un mélange de caroténoïdes et d'anti-oxydants et/ou de vitamines. Cette composition est destinée notamment à diminuer les radicaux fibres générés par l'irradiation U.V.
EP 659 402 concerne des formulations contenant une association d'antioxydants lipophiles et d'antioxydants hydrophiles, et plus spécifiquement des associations de polyphénols, en particulier provenant de Vitis vinifera, avec des caroténoïdes et/ou procaroténoïdes. Ces compositions ont une activité anti-radicalaire améliorée.

Ainsi, l'invention a pour objet premier l'utilisation du lycopène dans une composition, le lycopène étant destiné à traiter, de manière préventive et/ou curative, les signes cutanés du vieillissement chrono-biologique.

Par signes cutanés du vieillissement on entend toutes modifications de l'aspect extérieur de la peau dues au vieillissement qu'il soit chronobiologique et/ou photo-induit, comme par exemple les rides et ridules, la peau flétrie, la peau molle, la peau amincie, le manque d'élasticité et/ou de tonus de la peau, mais également toutes modifications internes de la peau qui ne se traduisent pas systématiquement par un aspect extérieur modifié, comme par exemple toutes dégradations internes de la peau, particulièrement du collagène, consécutives à une exposition aux rayonnements ultra-violets.

L'invention a pour second objet l'utilisation du lycopène dans une composition, le lycopène étant destiné à inhiber l'expression des protéases de la matrice extracellulaire, particulièrement des métalloprotéinases et encore plus particulièrement de la métalloprotéinase de type 1.

L'invention a pour troisième objet l'utilisation du lycopène dans une composition, le lycopène étant destiné à traiter les atteintes cutanées liées à la ménopause.

L'invention a pour quatrième objet l'utilisation du lycopène dans une composition, le lycopène étant destiné à lutter contre les rides et ridules.

L'invention a pour cinquième objet l'utilisation du lycopène dans une composition, le lycopène étant destiné à lutter contre la peau flétrie.

L'invention a pour sixième objet l'utilisation du lycopène dans une composition, le lycopène étant destiné à lutter contre la peau molle.

L'invention a pour septième objet l'utilisation du lycopène dans une composition, le lycopène étant destiné à lutter contre la peau amincie.

L'invention a pour huitième objet l'utilisation du lycopène dans une composition, le lycopène étant destiné à lutter contre le manque d'élasticité et/ou de tonus de la peau.

Le lycopène utilisé selon l'invention peut être d'origine naturelle ou synthétique. Par origine naturelle, on entend le lycopène, à l'état pur ou en solution quelle qu'en soit sa concentration dans ladite solution, obtenu à partir d'un élément naturel comme par exemple un extrait végétal, particulièrement la tomate. Par origine synthétique, on entend le lycopène, à l'état pur ou en solution quelle qu'en soit sa concentration dans ladite solution, obtenu par synthèse chimique.

Lorsque le lycopène est d'origine naturelle, il peut être obtenu à partir de matériel végétal issu de plante entière cultivée *in vivo* ou issu de culture *in vitro.*

Par cultivée *in vivo* on entend toute culture de type classique c'est à dire en sol à l'air libre ou en serre, ou encore hors sol.

Par culture *in vitro*, on entend l'ensemble des techniques connues de l'homme du métier qui permet de manière artificielle l'obtention d'un végétal ou d'une partie d'un végétal. La pression de sélection imposée par les conditions physico-chimiques lors de la croissance des cellules végétales *in vitro* permet d'obtenir un matériel végétal standardisé et disponible tout au long de l'année contrairement aux plantes cultivées *in vivo*.

Préférentiellement selon l'invention, on utilise un végétal issu de culture *in vivo*. Très préférentiellement selon l'invention, on utilise un extrait de tomate riche en lycopène.

Toute méthode d'extraction connue de l'homme du métier peut être utilisée pour préparer le lycopène utilisé selon l'invention.

Le lycopène peut être en suspension aqueuse ou en solution alcoolique, notamment éthanolique.
Par suspension aqueuse on entend une solution constituée totalement ou pour part d'eau. On peut citer ainsi l'eau elle-même, les solutions hydroalcooliques en toute proportion ou encore les solutions constituées d'eau et d'un composé comme le propylène glycol en toute proportion.

A titre d'exemple, selon l'invention on utilise un extrait de tomate riche en lycopène, préparé par la société Métaphar, commercialisé sous la dénomination LycOMato® constitué d'un extrait d'oléorésine contenant 6% de lycopène pur.

L'extrait peut constituer à lui seul le principe actif des compositions de l'invention.

La quantité d'extrait utilisable selon l'invention est bien entendu fonction de l'effet recherché et peut donc varier dans une large mesure.

Pour donner un ordre de grandeur, on peut utiliser le lycopène à l'état pur en une quantité représentant de 10⁻¹² % à 20% du poids total de la composition et préférentiellement en une quantité représentant de 10⁻⁸ % à 10 % du poids total de la composition.

Bien entendu l'homme du métier, s'il utilise le lycopène sous la forme d'une solution, un extrait végétal par exemple, sait ajuster la quantité de solution qu'il utilise dans sa composition afin que la quantité finale de lycopène dans la composition soit en accord avec les quantités utilisables précédemment définies.

La composition de l'invention peut être sous toutes formes galéniques imaginables, adaptées aussi bien à une application topique sur la peau et/ou les muqueuses et/ou les cheveux qu'à une administration par la voie orale. De manière préférentielle, la composition de l'invention est destinée à une administration par la voie orale.

La composition de l'invention est une composition cosmétique car elle est destinée à améliorer l'aspect cutané général de l'individu qui en fait usage.
Très préférentiellement la composition de l'invention est une composition cosmétique destinée à une administration par la voie orale.

Pour une administration par la voie orale, la composition de l'invention peut se présenter sous toutes les formes adaptées, particulièrement sous forme d'une solution buvable, d'un comprimé, d'une gélule , d'une capsule ou encore d'un aliment nutritionnel ou d'un complément nutritionnel.

Ladite composition comprend en outre au moins un excipient approprié adapté à l'administration orale.

Pour une administration par application topique sur la peau, les cheveux et/ou les muqueuses, la composition selon l'invention comprend bien évidemment un support cosmétiquement acceptable, c'est à dire un support compatible avec la peau, les muqueuses, les ongles, les cheveux et peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou mieux des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick. Elle peut être utilisée comme produit de soin, comme produit de nettoyage, comme produit de maquillage ou encore comme simple produit déodorant.

De façon connue, la composition de l'invention peut contenir les adjuvants habituels dans les domaines cosmétiques et dermatologique, tels que les gélifiants hydrophiles ou lipophiles, les actifs hydrophiles ou lipophiles, les conservateurs, les antioxydants, les solvants, les parfums, les charges, les filtres, les pigments, les agents chélateurs, les absorbeurs d'odeur et les matières colorantes. Les quantités de ces différents adjuvants sont celles classiquement utilisées dans les domaines considérés, et par exemple de 0,01% à 20 % du poids total de la composition. Ces adjuvants, selon leur nature, peuvent être introduits dans la phase grasse, dans la phase aqueuse, dans les vésicules lipidiques et/ou dans les nanoparticules.

Lorsque la composition de l'invention est une émulsion, la proportion de la phase grasse peut aller de 5 % à 80 % en poids, et de préférence de 5 % à 50 % du poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 % à 30 % en poids, et de préférence de 0,5 % à 20 % du poids total de la composition.

Comme huiles utilisables dans l'invention, on peut citer les huiles minérales, les huiles d'origine végétale (huile d'abricot, huile de tournesol), les huiles d'origine animale, les huiles de synthèse, les huiles siliconées et les huiles fluorées (perfluoropolyéthers). On peut aussi utiliser comme matières grasses des alcools gras (alcool cétylique), des acides gras, des cires (cire d'abeilles).

Comme émulsionnants et coémulsionnants utilisables dans l'invention, on peut citer par exemple les esters d'acide gras et de polyéthylène glycol tels que le stéarate de PEG-40, le stéarate de PEG-100, les esters d'acide gras et de polyol tels que le stéarate de glycéryle et le tristéarate de sorbitane.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

La présente invention a en outre pour objet un procédé de traitement cosmétique de la peau destiné à stimuler la synthèse du collagène et/ou lutter contre les atteintes cutanées liées à l'âge et/ou à la ménopause et/ou lutter contre l'amincissement du derme et/ou combattre l'apparence de la peau molle et/ou ridée, caractérisé par le fait que l'on applique sur la peau, sur les cheveux, et/ou sur les muqueuses, ou que l'on ingère une composition cosmétique comprenant au moins du lycopène.

Le procédé de traitement cosmétique de l'invention peut être mis en oeuvre notamment en appliquant les compositions cosmétiques telles que définies ci-dessus, selon la technique d'utilisation habituelle de ces compositions. Par exemple : application de crèmes, de gels, de sérums, de lotions, de laits, de shampooings ou de compositions anti-solaires, sur la peau, ou sur les cheveux ou encore application de dentifrice sur les gencives et de manière préférentielle par administration par voie orale d'une solution buvable, d'un sirop, d'un comprimé, d'une gélule ou d'une capsule ou encore d'un aliment nutritionnel ou d'un complément nutritionnel.

Les exemples et compositions suivants illustrent l'invention sans la limiter aucunement. Dans les compositions les proportions indiquées sont des pourcentages en poids.

### Exemple 1 : Evaluation de l'activité du lycopène sur les collagénases interstitielles :

L'activité d'un extrait de tomate riche en lycopène (LycOMato® de la société Metaphar) a été étudiée sur des cellules HeLa transfectées avec un vecteur d'expression codant pour une partie du promoteur de la collagénase cloné en amont du gène de la chloramphénicol acétyltransférase (CAT).
L'étude permet d'évaluer l'efficacité d'un produit à inhiber l'induction de la transcription du gène de la collagénase par le Phorbol 12 Myristate 13 Acétate (PMA).

Le test est réalisé sur des cellules HeLa (ATCC CCL2) dans lesquelles le promoteur du gène de la collagénase 1 (3,8 kB) cloné en amont du gène de la chloramphénicol acétyltransférase (CAT) a été introduit.
Les cellules sont exposées au PMA à la concentration de 10 nM pendant 24 heures
Avant l'administration de PMA, les cellules ont été mises en contact pendant 2 heures avec le lycopène (LycOMato® de la société Metaphar) à des concentrations variant de 10⁻⁷ % à 10⁻⁴ %, représentant des concentrations en lycopène allant de 6.10⁻⁹ % à 6.10⁻⁶ %.

Le dosage de la CAT est réalisé à l'aide d'un kit "CAT-Elisa" vendu par la société Boehringer, selon les indications du fournisseur.

Un "taux de base" de l'expression de la CAT est mesuré dans des cellules n'ayant reçu ni PMA, ni traitement par le produit à tester.

Les résultats du test, présentés dans le tableau suiuvant, sont exprimés par rapport au taux de base (100%), en pourcentage d'inhibition de l'expression du gène CAT.
Ces résultats reflètent donc l'activité du promoteur de la collagénase 1 après induction par le PMA et en présence ou non du produit à tester.

| | | | | | |
|---|---|---|---|---|---|
| LycOMato® (en %) | 0 | 10⁻⁷ | 10⁻⁶ | 10⁻⁵ | 10⁻⁴ |
| % d'inhibition de l'activité du promoteur | 0 | 15 | 25 | 40 | 50 |

Ces résultats montrent bien l'effet inhibiteur du lycopène sur la synthèse de la collagénase 1, par inhibition de l'activité de son promoteur.

### Exemple 2 : Exemples de formulations illustrant l'invention et particulièrement les compositions selon l'invention. Ces compositions ont été obtenues par simple mélange des différents composants.

### Composition 1 - Capsules moiles :

### Excipients :

| | |
|---|---|
| Huile de Soja | 40 mg |
| Huile de Germe de Blé | 85 mg |
| Lécithines de Soja | 25 mg |

### Vitamine :

| | |
|---|---|
| Tocopherols naturels | 3 mg |

### Composants :

| | |
|---|---|
| Lycopène 6 % | 175 mg |

### Composition 2 : Shampooing

| | |
|---|---|
| Lycopène | 10⁻⁷ % |
| Hydroxypropylcellulose (Klucel H® vendu par la société Hercules) | 1,00 % |
| Parfum | 0,50 % |
| Conservateur | 0,30 % |
| Eau | qsp 100,00 % |

### Composition 3 crème de soin du visage (émulsion huile dans eau)

| | |
|---|---|
| Lycopène | 0.01 % |
| Stéarate de glycérol | 2,00 % |
| Polysorbate 60 (Tween 60® vendu par la société ICI) | 1,00 % |
| Acide stéarique | 1,40 % |
| Triéthanolamine | 0,70 % |
| Carbomer | 0,40 % |
| Fraction liquide du beurre de karité | 12,00 % |
| Perhydrosqualène | 12,00 % |
| Antioxydant | 0,05 % |
| Parfum | 0,50 % |
| Conservateur | 0,30 % |
| Eau | qsp 100,00 % |

### Composition 4 : Gel pour la peau

| | |
|---|---|
| Lycopène | 0.01 % |
| Acide tout trans rétinoïque | 0,05 % |
| Hydroxypropylcellulose (Klucel H®vendu par la société Hercules) | 1,00 % |
| Antioxydant | 0,05 % |
| Isopropanol | 40,00 % |
| Conservateur | 0,30 % |
| Eau | qsp 100,00 % |

### Composition 5 : Gel pour le soin du visage

| | |
|---|---|
| Lycopène | 10⁻⁵ % |
| Hydroxypropylcellulose (Klucel H® vendu par la société Hercules) | 1,00 % |
| Antioxydant | 0,05 % |
| Isopropanol | 40,00 % |
| Conservateur | 0,30 % |
| Eau | qsp 100,00 % |

### Composition 6 : Gel

| | |
|---|---|
| Lycopène | 0.10 % |
| Hydroxypropylcellulose (Klucel H® vendu par la société Hercules) | 1,00 % |
| Antioxydant | 0,05 % |
| Chlorhydrate de lidocaïne | 2,00 % |
| Isopropanol | 40,00 % |
| Conservateur | 0,30 % |
| Eau | qsp 100,00 % |

### Composition 7 : Crème de soin de l'érythème solaire (émulsion huile-dans-eau)

| | |
|---|---|
| Lycopène | 10⁻⁶ % |
| Stéarate de glycérol | 2,00 % |
| Polysorbate 60 (Tween 60® vendu par la société ICI) | 1,00 % |
| Acide stéarique | 1,40 % |
| Acide glycyrrhétinique | 2,00 % |
| Triéthanolamine | 0,70 % |
| Carbomer | 0,40 % |
| Fraction liquide du beurre de karité | 12,00 % |
| Huile de tournesol | 10,00 % |
| Antioxydant | 0,05 % |
| Parfum | 0,50 % |
| Conservateur | 0,30 % |
| Eau | qsp 100,00 % |

### Composition 8 : Crème de soin antirides pour le visage (émulsion huile/eau)

| | |
|---|---|
| Lycopène | 10⁻⁷% |
| Stéarate de glycérol | 2,00 % |
| Polysorbate 60 (Tween 60® vendu par la société ICI) | 1,00 % |
| Acide stéarique | 1,40 % |
| Acide n-octanoyl-5-salicylique | 0,50 % |
| Triéthanolamine | 0,70 % |
| Carbomer | 0,40 % |
| Fraction liquide du beurre de karité | 12,00 % |
| Perhydrosqualène | 12,00 % |
| Antioxydant | 0,05 % |
| Parfum | 0,50 % |
| Conservateur | 0,30 % |
| Eau | qsp 100,00 % |

### Composition 9 : Lotion

| | |
|---|---|
| Lycopène | 1,00 % |
| Acide glycolique | 50,00 % |
| Hydroxypropylcellulose (Klucel H® vendu par la société Hercules) | 0,05 % |
| Conservateur | 0,30 % |
| NaOH | qsp pH = 2,8 |
| Ethanol | qsp 100,00 % |

### Composition 10 : Lotion démaquillante pour le visage

| | |
|---|---|
| Lycopène | 10⁻⁴ % |
| Antioxydant | 0,05 % |
| Isopropanol | 40,00 % |
| Conservateur | 0,30 % |
| Eau | qsp 100,00 % |

## Revendications

1. Utilisation, dans une composition, du lycopène comme agent pour traiter, de manière préventive et/ou curative, les signes cutanés du vieillissement chronobiologique.

2. Utilisation du lycopène selon la revendication 1, le lycopène étant destiné à inhiber l'expression des protéases de la matrice extracellulaire.

3. Utilisation selon l'une au moins des revendications 1 ou 2, **caractérisée par le fait que** le lycopène est destiné à inhiber l'expression des métalloprotéinases.

4. Utilisation selon l'une quelconque des revendications 1 à 3, **caractérisée par le fait que** le lycopène est destiné à inhiber l'expression de la métalloprotéinase de type 1.

5. Utilisation du lycopène dans une composition selon l'une au moins des revendications 1 à 4, le lycopène étant destiné à traiter les atteintes cutanées liées à la ménopause.

6. Utilisation du lycopène dans une composition selon l'une au moins des revendications 1 à 4, le lycopène étant destiné à lutter contre les rides et ridules.

7. Utilisation du lycopène dans une composition selon l'une au moins des revendications 1 à 4, le lycopène étant destiné, à lutter contre la peau flétrie.

8. Utilisation du lycopène dans une composition selon l'une au moins des revendications 1 à 4, le lycopène étant destiné à lutter contre la peau molle.

9. Utilisation du lycopène dans une composition selon l'une au moins des revendications 1 à 4, le lycopène étant destiné à lutter contre la peau amincie.

10. Utilisation du lycopène dans une composition selon l'une au moins des revendications 1 à 4, le lycopène étant destiné à lutter contre le manque d'élasticité et/ou de tonus de la peau.

11. Utilisation du lycopène dans une composition selon l'une au moins des revendications 1 à 10 , le lycopène étant destiné à lutter contre les dégradations du collagène.

12. Utilisation selon l'une quelconque des revendications précédentes, **caractérisée par le fait que** le lycopène est d'origine naturelle ou synthétique.

13. Utilisation selon l'une quelconque des revendications précédentes, **caractérisée par le fait que** le lycopène est sous forme purifiée ou sous la forme d'une solution.

14. Utilisation selon la revendication 13, **caractérisée par le fait que** la solution est un extrait de végétal.

15. Utilisation selon la revendication 14, **caractérisée par le fait que** l'extrait de végétal est un extrait de tomate.

16. Utilisation selon la revendication 15, **caractérisée par le fait que** l'extrait est un extrait d'oléorésine de tomate contenant 6% de lycopène.

17. Utilisation selon l'une quelconque des revendications précédentes, **caractérisée par le fait que** le lycopène est utilisé en une quantité représentant de 10⁻¹² % à 20 % du poids total de la composition.

18. Utilisation selon l'une quelconque des revendications précédentes, **caractérisée par le fait que** le lycopène est utilisé en une quantité représentant de 10⁻⁸ % à 10 % du poids total de la composition.

19. Utilisation selon l'une quelconque des revendications précédentes, dans une composition destinée à une administration par la voie orale.

20. Utilisation selon la revendication précédente, **caractérisé par le fait que** la composition se présente sous la forme d'une solution buvable, d'un sirop, d'un comprimé, d'une gélule, d'une capsule ou encore d'un aliment nutritionnel ou d'une complément nutritionnel.

21. Utilisation cosmétique de lycopène pour lutter contre les atteintes cutanées liées à l'âge et/ou à la ménopause et/ou lutter contre l'amincissement du derme et/ou combattre l'apparence de la peau molle et ridée, dans laquelle on applique sur la peau, sur les cheveux, et/ou sur les muqueuses, ou on ingère une composition cosmétique comprenant au moins du lycopène.

## Patentansprüche

1. Verwendung von Lycopin in einer Zusammensetzung als Mittel für die präventive und/oder kurative Behandlung der Anzeichen der chronobiologischen Alterung der Haut.

2. Verwendung von Lycopin nach Anspruch 1, wobei das Lycopin dazu vorgesehen ist, die Expression von Proteasen der extrazellulären Matrix zu inhibieren.

3. Verwendung nach einem der Ansprüche 1 oder 2, **dadurch gekennzeichnet, dass** das Lycopin dazu vorgesehen ist, die Expression von Metalloproteinasen zu inhibieren.

4. Verwendung nach einem der Ansprüche 1 bis 3, **dadurch gekennzeichnet, dass** das Lycopin dazu vorgesehen ist, die Expression der Metalloproteinase vom Typ 1 zu inhibieren.

5. Verwendung des Lycopins in einer Zusammensetzung nach mindestens einem der Ansprüche 1 bis 4, wobei das Lycopin dazu vorgesehen ist, die mit der Menopause zusammenhängenden Beeinträchtigungen der Haut zu behandeln.

6. Verwendung des Lycopins in einer Zusammensetzung nach mindestens einem der Ansprüche 1 bis 4, wobei das Lycopin dazu vorgesehen ist, Falten und Fältchen zu bekämpfen.

7. Verwendung des Lycopins in einer Zusammensetzung nach mindestens einem der Ansprüche 1 bis 4, wobei das Lycopin dazu vorgesehen ist, schlaffe Haut zu bekämpfen.

8. Verwendung des Lycopins in einer Zusammensetzung nach mindestens einem der Ansprüche 1 bis 4, wobei das Lycopin dazu vorgesehen ist, weiche Haut zu bekämpfen.

9. Verwendung des Lycopins in einer Zusammensetzung nach mindestens einem der Ansprüche 1 bis 4, wobei das Lycopin dazu vorgesehen ist, dünner gewordene Haut zu bekämpfen.

10. Verwendung des Lycopins in einer Zusammensetzung nach mindestens einem der Ansprüche 1 bis 4, wobei das Lycopin dazu vorgesehen ist, Elastizitätsmangel und/oder mangelnden Tonus der Haut zu bekämpfen.

11. Verwendung des Lycopins in einer Zusammensetzung nach mindestens einem der Ansprüche 1 bis 10, wobei das Lycopin dazu vorgesehen ist, die Zerstörung des Collagens zu bekämpfen.

12. Verwendung nach einem der vorhergehenden Ansprüche, **dadurch gekennzeichnet, dass** das Lycopin natürlicher oder synthetischer Herkunft ist.

13. Verwendung nach einem der vorhergehenden Ansprüche, **dadurch gekennzeichnet, dass** das Lycopin in reiner Form oder in Form einer Lösung vorliegt.

14. Verwendung nach Anspruch 13, **dadurch gekennzeichnet, dass** die Lösung ein Pflanzenextrakt ist.

15. Verwendung nach Anspruch 14, **dadurch gekennzeichnet, dass** der Pflanzenextrakt ein Tomatenextrakt ist.

16. Verwendung nach Anspruch 15, **dadurch gekennzeichnet, dass** der Extrakt ein Oleoresin-Extrakt aus Tomaten mit 6 % Lycopin ist.

17. Verwendung nach einem der vorhergehenden Ansprüche, **dadurch gekennzeichnet, dass** das Lycopin in einer Menge verwendet wird, die 10⁻¹² bis 20 % des Gesamtgewichts der Zusammensetzung ausmacht.

18. Verwendung nach einem der vorhergehenden Ansprüche, **dadurch gekennzeichnet, dass** das Lycopin in einer Menge von 10⁻⁸ bis 10 % des Gesamtgewichts der Zusammensetzung verwendet wird.

19. Verwendung nach einem der vorhergehenden Ansprüche in einer Zusammensetzung, die für eine Verabreichung auf oralem Weg vorgesehen ist.

20. Verwendung nach dem vorhergehenden Anspruch, **dadurch gekennzeichnet, dass** die Zusammensetzung als Trinklösung, Sirup, Tablette, Gelatinekapsel, Kapsel oder Nahrungsmittel oder Nahrungsergänzungsmittel vorliegt.

21. Kosmetische Verwendung von Lycopin zur Bekämpfung von Beeinträchtigungen der Haut, die mit dem Alter und/oder der Menopause zusammenhängen, und/oder zur Bekämpfung des Dünnerwerdens der Dermis und/oder zur Bekämpfung des Auftretens von weicher und faltiger Haut, wobei auf die Haut, die Haare und/oder die Schleimhäute eine kosmetische Zusammensetzung, die zumindest Lycopin enthält, aufgetragen wird oder eine solche Zusammensetzung eingenommen wird.

## Claims

1. Use, in a composition, of lycopene as agent for treating, preventively and/or curatively, cutaneous signs of chronobiological ageing.

2. Use of lycopene according to Claim 1, the lycopene being intended to inhibit the expression of the proteases of the extracellular matrix.

3. Use according to at least one of Claims 1 and 2, **characterized in that** the lycopene is intended to inhibit the expression of metalloproteinases.

4. Use according to any one of Claims 1 to 3, **characterized in that** the lycopene is intended to inhibit the expression of type 1 metalloproteinase.

5. Use of lycopene in a composition according to at least one of Claims 1 to 4, the lycopene being intended to treat cutaneous conditions related to the menopause.

6. Use of lycopene in a composition according to at least one of Claims 1 to 4, the lycopene being intended to combat wrinkles and fine lines.

7. Use of lycopene in a composition according to at least one of Claims 1 to 4, the lycopene being intended to combat withered skin.

8. Use of lycopene in a composition according to at least one of Claims 1 to 4, the lycopene being intended to combat flabby skin.

9. Use of lycopene in a composition according to at least one of Claims 1 to 4, the lycopene being intended to combat thinned skin.

10. Use of lycopene in a composition according to at least one of Claims 1 to 4, the lycopene being intended to combat lack of elasticity and/or of tone of the skin.

11. Use of lycopene in a composition according to at least one of Claims 1 to 10, the lycopene being intended to combat damage to the collagen.

12. Use according to any one of the preceding claims, **characterized in that** the lycopene is natural or synthetic in origin.

13. Use according to any one of the preceding claims, **characterized in that** the lycopene is in the purified form or in the form of a solution.

14. Use according to Claim 13, **characterized in that** the solution is a plant extract.

15. Use according to Claim 14, **characterized in that** the plant extract is a tomato extract.

16. Use according to Claim 15, **characterized in that** the extract is a tomato oleoresin extract comprising 6% of lycopene.

17. Use according to any one of the preceding claims, **characterized in that** the lycopene is used in an amount representing from 10⁻¹²% to 20% of the total weight of the composition.

18. Use according to any one of the preceding claims, **characterized in that** the lycopene is used in an amount representing from 10⁻⁸% to 10% of the total weight of the composition.

19. Use according to any one of the preceding claims, in a composition intended for oral administration.

20. Use according to the preceding claim, **characterized in that** the composition is provided in the form of a solution to be taken orally, of a syrup, of a tablet, of a capsule, including a hard gelatin capsule, of a nutritional food or of a nutritional supplement.

21. Cosmetic use of lycopene to combat cutaneous conditions related to age and/or to the menopause and/or to combat thinning of the dermis and/or to combat the appearance of flabby and wrinkled skin, in which a cosmetic composition comprising at least lycopene is applied to the skin, to the hair and/or to the mucous membranes or is ingested.
